# EUROPEAN PATENT SPECIFICATION

(11) **EP 3 419 609 B1**
(45) Date of publication and mention of the grant of the patent: **26.04.2023**
(21) Application number: 17706788.1
(22) Date of filing: 23.02.2017
(51) Int. Cl.: A61K 31/122, A61K 31/216, A61K 31/42, A61P 3/00

(54) **TREATMENT OF ALKAPTONURIA AND TYROSINEMIA TYPE 1**
BEHANDLUNG VON ALKAPTONURIE UND TYROSINEMIE TYP 1
TRAITEMENT DE L'ALCAPTONURIE ET DE LA TYROSINÉMIE DE TYPE 1

(30) Priority: 23.02.2016 IT UB20160972
(43) Date of publication of application: 02.01.2019
(73) Proprietor: Università degli Studi di Siena, 53100 Siena (IT)
(72) Inventor: SANTUCCI, Annalisa, 53100 Siena (SI) (IT); BERNARDINI, Giulia, 53100 Siena (SI) (IT); LASCHI, Marcella, 53100 Siena (SI) (IT); MANETTI, Fabrizio, 53100 Siena (SI) (IT); PETRICCI, Elena, 53100 Siena (SI) (IT)
(74) Representative: Capasso, Olga
(86) International application number: PCT/EP2017/054158
(87) International publication number: WO 2017/144583

(56) References cited:
- WO-A1-2012/177214
- US-A- 4 780 127
- WENDY J INTRONE ET AL: "A 3-year randomized therapeutic trial of nitisinone in alkaptonuria", MOLECULAR GENETICS AND METABOLISM, ACADEMIC PRESS, AMSTERDAM, NL, vol. 103, no. 4, 28 April 2011 (2011-04-28), pages 307-314, XP028249729, ISSN: 1096-7192, DOI: 10.1016/J.YMGME.2011.04.016 [retrieved on 2011-05-06]
- CHANIKA PHORNPHUTKUL ET AL: "Natural History of Alkaptonuria", NEW ENGLAND JOURNAL OF MEDICINE, vol. 347, no. 26, 26 December 2002 (2002-12-26), pages 2111-2121, XP055212052, ISSN: 0028-4793, DOI: 10.1056/NEJMoa021736
- SCHLUNE A ET AL: "Single dose NTBC-treatment of hereditary tyrosinemia type I", JOURNAL OF INHERITED METABOLIC DISEASE, KLUWER ACADEMIC PUBLISHERS, DO, vol. 35, no. 5, 4 February 2012 (2012-02-04), pages 831-836, XP035106005, ISSN: 1573-2665, DOI: 10.1007/S10545-012-9450-9
- XU YU-LING ET AL: "Pyrazolone-quinazolone hybrids: A novel class of human 4-hydroxyphenylpyruvate dioxygenase inhibi", BIOORGANIC & MEDICINAL CHEMISTRY, PERGAMON, GB, vol. 22, no. 19, 18 August 2014 (2014-08-18), pages 5194-5211, XP029061992, ISSN: 0968-0896, DOI: 10.1016/J.BMC.2014.08.011
- LASCHI MARCELLA ET AL: "Inhibition of para-Hydroxyphenylpyruvate Dioxygenase by Analogues of the Herbicide Nitisinone As a Strategy to Decrease Homogentisic Acid Levels, the Causative Agent of Alkaptonuria.", CHEMMEDCHEM 5 APR 2016, vol. 11, no. 7, 5 April 2016 (2016-04-05), pages 674-678, XP002762816, ISSN: 1860-7187

## Description

### TECHNICAL FIELD

The present invention relates to a compound of formula I or II for use in the prevention and/or treatment of a disease characterized by accumulation of homogentisic acid (HGA) or of succinyl acetoacetate and succinylacetone:

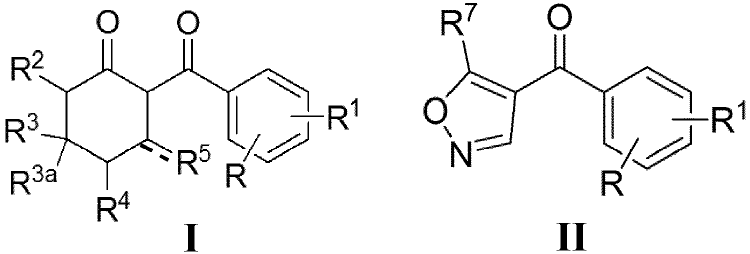

The present invention also relates to compositions comprising such compounds.

### BACKGROUND ART

Alkaptonuria (AKU; OMIM no. 203500) is a rare genetic disease characterized by a highly debilitating multi-systemic metabolic disorder caused by an accumulation of homogentisic acid (HGA) due to insufficient activity of the enzyme homogentisate 1,2-dioxygenase (HGD) in the catabolic pathway of tyrosine. HGA is oxidized, in a process called ochronosis, to a melanin-like polymeric pigment that deposits in the connective tissues, especially in cartilage, resulting in early and severe arthritis, heart damage and elevated motor disability [1, 2]. Experimental evidence shows that HGD is also expressed at the cellular level of the osteoarticular compartment where the disease is mainly localized, thus contributing to the induction of local ochronosis in alkaptonuric arthropathy [3].

As of today, no approved pharmacological cure exists for AKU although many therapeutic approaches have been attempted which have however proved to be palliative since they do not address the intrinsic cause of the illness [4, 5].

The only drug with some beneficial effect is the herbicide 2-(2-nitro-4-(trifluoromethyl)benzoyl)cyclohexane-1,3-dione, called Nitisinone or NTBC **(1)** [6]. Compound **1** is a potent inhibitor of the human and rat 4-hydroxyphenylpyruvate dioxygenase (4-HPPD) enzyme which is an Fe(II)-dependent non-heme oxygenase of crucial importance in tyrosine catabolism. Since 4-HPPD catalyses the transformation of the 4-hydroxyphenylpyruvate (HPP) to HGA [7], compound **1** should prevent or at least slow down the progression of the disease by reducing the accumulation of HGA.

The use of compound **1** has been approved by the FDA "in exceptional circumstances" as a life-saver in hereditary tyrosinemia type 1 (HT1), a rare paediatric disease wherein the biochemical abnormality lies in the deficiency of the fumarylacetoacetate hydrolase (FAH) enzyme in the tyrosine catabolic pathway. Consequently, there is an accumulation of the toxic metabolites, succinyl acetoacetate and succinylacetone, which cause severe renal and hepatic dysfunction, neurological crises and a high risk of developing hepatocellular carcinoma that quickly leads to death if a liver transplant is not performed [8]. Fumarylacetoacetate hydrolase acts on tyrosine after 4-HPPD does. Even though the treatment of HT1 with compound **1** leads to a clinical improvement, the percentage of patients who develop hepatocellular carcinoma remains high. In addition, serious side effects have been observed such as visual disorders, liver damage, convulsions, cognitive and learning difficulties associated with chronically high levels of tyrosine in the plasma caused by blocking of the tyrosine catabolism [8, 9, 10, 11].

Preclinical toxicological and pharmacokinetic data on compound **1** derive mainly from studies conducted in different animal models [12-14] and, although in recent years some toxicological and pharmacokinetic data have also been obtained on AKU and HT1 patients [4, 5, 8-11, 15], to date the safety margins, especially in the long term, of use of compound **1** in humans cannot be fully determined [4, 9, 10].

However, the theoretical rationale for using compound **1** in AKU has been experimentally confirmed in terms of the reduction in plasma HGA; in fact, the four clinical trials which have been conducted on AKU (https://clinicaltrials.gov/ct2/show/NCT00107783, https://clinicaltrials.gov/ct2/show/NCT01390077, https://clinicaltrials.gov/ct2/show/NCT01828463, SONIA1; https://clinicaltrials.gov/ct2/show/NCT01916382, SONIA2) [4, 15-18] have shown that compound **1** consistently reduces HGA despite causing a significant accumulation of tyrosine in the plasma. In the clinical trial SONIA1 [4, 18], undertaken by the DevelopAKUre Consortium and aimed at obtaining a marketing authorization for compound **1** in the treatment of AKU from the European Medecine Agency (EMA), it was shown that compound **1** reduces the urinary excretion of HGA in a dose-dependent manner while increased plasma tyrosine levels are not clearly correlated with the concentration of compound **1.** Moreover, again in this study, several pharmacokinetic parameters of compound **1** were determined, such as the area under the curve, the maximum concentration and the oral clearance [4, 18]. However the clinical and physio-pathological significance of the increase in tyrosine due to treatment with compound **1** is not yet fully known. Then, long-term monitoring of tyrosinemia is required to ensure a safe use of compound **1** [19, 20]. In fact, even lower doses of compound **1** induce levels of tyrosinemia exceeding the recommended threshold of 400-500 µmol/L [4, 18, 20]. Wendy J Introne et al (Molecular Genetics and Metabolism, Academic Press, Amsterdam NL, vol 103, no. 4, 28 April 2011, pages 307-314) discloses that a 3-year randomized therapeutic trial of Nitisinone in alkaptonuria shows that biochemically this study consistently demonstrated 95% reduction of HGA in urine and plasma over the course of 3 years. Chanika Phornphutkul et al (New England Journal of Medicine, vol 347, no. 26, 2002, pages 2111-2121) discloses that alkaptonuria, caused by mutations in the HGO gene and a deficiency of homogentisate 1,2-dioxygenase, results in an accumulation of homogentisic acid (HGA), ochronosis, and destruction of connective tissue. Although Nitisinone can reduce HGA production in humans with homogentisate 1,2-dioxygenase deficiency, the long-term safety and efficacy of this treatment require further evaluation. Schlune et al (Journal of inherited Metabolic Disease, vol 35, no. 5, 4 February 2012, pages 831-836) discloses that a single daily dose NTBC-treatment of hereditary tyrosinemia type I (NTBC: 2-(2-nitro-4-trifluoromethylbenzoyl)-1,3-cyclohexandione) is the mainstay of treatment in tyrosinemia type 1 (HT1). WO 2012/177214 discloses nitisinone for treating a medical condition selected from tyrosinemia, Parkinson's disease, depression, restless leg syndrome and alkaptonuria. Xu Yu-Ling et al (Bioorganic & Medicinal Chemistry, vol 22, no. 19, 2014, pages 5194-5211) discloses alternative compounds and nitisinone testing on 4-hydroxyphenylpyruvate dioxygenase in relation to plant physiology but also alkaptonuria. US 4,780,127 discloses the compound 5,5-dimethyl-2-[2-nitro-4-(trifluoromethyl)benzoyl]-1,3-cyclohexanedione and herbicidal compositions containing the compound.

In this context, it is important to obtain complete preclinical data on compound **1,** particularly at the human cellular level. Further, there is the need for alternative 4-HPPD inhibitors which are able to significantly reduce HGA while minimizing tyrosinemia, for a promising treatment of AKU and HT1.

### SUMMARY OF THE INVENTION

In the present invention, a commercial library was analysed to identify 4-HPPD inhibitors. Various compounds were found to inhibit 4-HPPD with an advantageous profile compared to compound 1, i.e. with better enzyme inhibition (IC₅₀), lower toxicity (LD₅₀) and lower tyrosine accumulation. These compounds are therefore advantageous and useful for the treatment of AKU and HT-1.

The present invention provides a compound of formula I or II for use in the prevention and/or treatment of a disease characterized by accumulation of homogentisic acid (HGA) or of succinyl acetoacetate and succinylacetone: 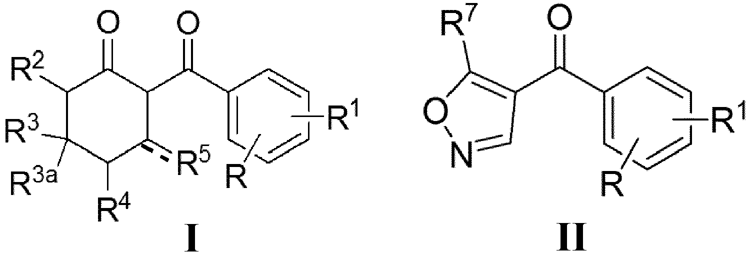 wherein
--- represents the presence or absence of a bond;
   - R and R¹ are identical or different and independent of each other, in ortho, meta or para position, and represent -H; -OH; -NH₂; -NO₂; -SO₂CH₃; a halogen atom; an alkyl group; a haloalkyl group; an alkoxy group -O-alkyl; a cycloalkyl group; a phenyl group or an aryl group;
   - R², R³, R^{3a}, R⁴ and R⁵ are identical or different and independent of each other, and represent - H; -OH; -NH₂; -NO₂; -SO₂CH₃; a halogen atom; an alkyl group; a carbonyl group, a cycloalkyl group, an alkoxy group; a phenyl group; an aryl group, or a heteroaryl group; an amide group - C(=O)-NH-R⁶ or an ester group -C(=O)-O-R⁶;
further, R⁵ may be oxo when the bond between R⁵ and the carbon to which R⁵ is attached is a double bond,
further, keeping the above defined substituents for R and R¹, the groups R² and R³ or R^{3a}, R³ or R^{3a} and R⁴, R⁴ and R⁵ may be fused to form, together with the carbon atoms adjacent to the cyclohexanone group on which they are located, a cycle with 5, 6 or 7 atoms condensed to the same cyclohexanone;
   - R⁶ represents an alkyl group, a cycloalkyl group, a phenyl group, an aryl group or a heteroaryl group;
   - R⁷ represents: -H; -OH; -NH₂; -NO₂; -SO₂CH₃; a halogen atom; an alkyl group; an alkoxy group; a cycloalkyl group; a phenyl group or an aryl group,
wherein the compound of formula I in which R is ortho-NO₂, R¹ is para-CF₃, R² = R³ = R^{3a} = R⁴ and is H and R⁵ is oxo when the bond between R⁵ and the carbon to which R⁵ is attached is a double bond (known as Nitisinone) is not included.

Preferably
- the halogen atom is Cl or Br and/or;
- the alkyl group comprises a carbon chain that contains from 1 to 10 carbon atoms, said chain being linear or branched, saturated or unsaturated, with one or more double bonds and/or one or more triple bonds, said chain being unsubstituted or substituted with one or more heteroatoms such as O, S, or N, or with one or more halogen atoms, or with one or more aryl or heteroaryl groups and/or;
- the haloalkyl group is CF₃, CHF₂, CH₂F and/or;
- the cycloalkyl group comprises from 3 to 8 carbon atoms, preferably the cycloalkyl group is cyclopropyl, cyclopentyl or cyclohexyl and/or;
- the heteroaryl group is pyrrole, furan, thiophene, oxazole, isoxazole, 3-methylisoxazole, pyrazole, imidazole or pyridine.

Preferably
- R is H, *ortho*-chloro or *ortho-*NO₂;
- R¹ is H, *para*-Cl*, para*-SO₂CH₃, *para*-NO₂, *para*-CF₃;
- R² is H, Cl, OH, phenyl, 3-methlylisoxazol-6-yl;
- R³ e R^{3a} are H or CH₃;
- R⁴ is H, OH, Cl, COOCH₃ and
- R⁵ is H or oxo when the bond between R⁵ and the carbon to which R⁵ is attached is a double bond.

Still preferably the compound is selected from the group consisting of:
- 2-[2-Chloro-4-methylsulfonyl-3-(2,2,2-trifluoroetoxyimethyl)benzoyl]-4,6-dihydroxy-cyclohexane-1,3-dione, compound **2:** 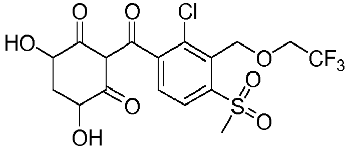
- 2-[2-Chloro-4-methylsulfonyl-3-(2,2,2-trifluoroetoxymethyl)benzoyl]cyclohexane-1,3-dione, known as tembotrione, compound **3:** 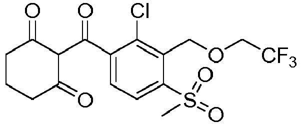
- 2-(4-Methylsulfonyl-2-nitrobenzoyl)-cyclohexane-1,3-dione, known as mesotrione, compound **4:** 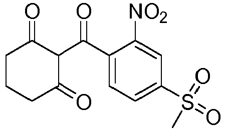
- 2-(2-chloro-4-nitro-benzoyl)-5,5-dimethyl-cyclohexane-1,3-dione, compound **6:** 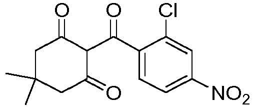
- 2,2-Dimethyl-5-(4-nitrobenzoyl)-4,6-dioxycyclohexane methyl carboxylate, compound **7:** 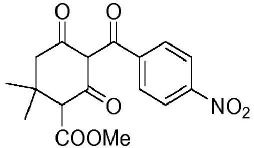
- 2-Benzoyl-4-chloro-5,5-dimethyl-cyclohexane-1,3-dione, compound **8:** 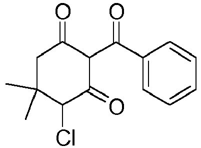
- 2-Benzoyl-5,5-dimethyl-cyclohexane-1,3-dione, compound **9:** 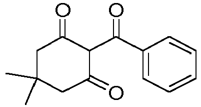
- 2-(4-chlorobenzoyl)-5,5-dimethyl-4-(3-methyl-isoxazol-5-yl)-cyclohexane-1,3-dione, compound **10:** 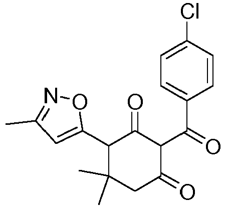
- 2-Benzoyl-4,6-dichloro-5,5-dimethyl-cyclohexane-1,3-dione, compound **11:** 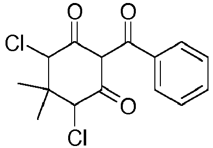
- 5-Benzoyl-2,2-dimethyl-4,6-dioxacyclohexane methyl carboxylate, compound **12:** 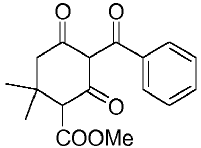
- 2- (2,4-dichlorobenzoyl) -4-phenyl-cyclohexane-1,3-dione, compound **13:** 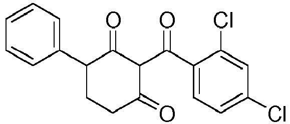
- 2-(2-Chloro-4-trifluoromethylbenzoyl)-5,5-dimethyl-cyclohexane-1,3-dione, compound **14:** 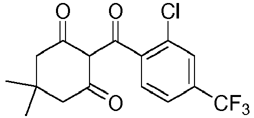
- 2-(2-Nitro-4-trifluoromethylbenzoyl)-5,5-dimethyl-cyclohexane-1,3-dione, compound **15:**

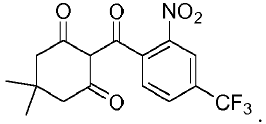

More preferably the compound is selected from the group consisting of:

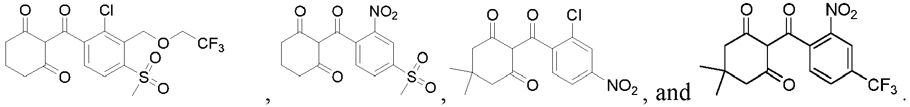

In a preferred embodiment the disease characterized by accumulation of HGA is alkaptonuria. In a preferred embodiment the disease characterized by accumulation of succinyl acetoacetate and succinylacetone is tyrosinemia type I.

Preferably the compound of formula I or II as above is used in combination with a therapeutic intervention.

Preferably the therapeutic intervention is selected from the group consisting of: low protein diet, administration of 4-HPPD inhibitor, anti-oxidants, pain killers, anti-inflammatory drugs, physiotherapy, joints replacement.

Still preferably the further therapeutic intervention is nitisinone.

The invention also provides a compound of formula I being

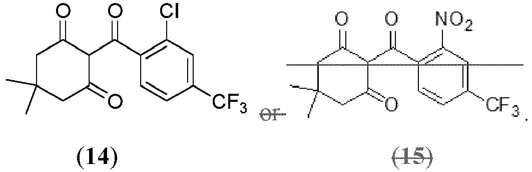

In a preferred embodiment the compound **14** is used in combination with a therapeutic intervention.

Preferably the therapeutic intervention is selected from the group consisting of: low protein diet, administration of 4-HPPD inhibitor, anti-oxidants, pain killers, anti-inflammatory drugs, physiotherapy, joints replacement.

The compounds of the invention may be combined to any therapeutic intervention known to be effective for the treatment and/or prevention of AKU and HT1.

In particular, the low protein diet reduces tyrosine and phenylalanine, the anti-oxidant may be ascorbic acid, pain killers and anti-inflammatory drugs are supporting therapies and may include paracetamol, any non-steroidal anti-inflammatory drugs.

Still preferably the further therapeutic intervention is nitisinone.

The present invention further provides a pharmaceutical composition comprising at least one compound **14** or **15** or a combination thereof.

The present invention further provides a pharmaceutical composition comprising at least one compound of formula I or II as defined above and a pharmaceutically acceptable excipient for use in the prevention and/or treatment of a disease characterized by accumulation of HGA or of succinyl acetoacetate and succinylacetone.

The pharmaceutical composition as defined above further comprising a therapeutic agent.

Preferably the therapeutic agent is selected from the group consisting of: 4-HPPD inhibitor, anti-oxidants, pain killers, anti-inflammatory drugs. In particular the anti-oxidant may be ascorbic acid, pain killers and anti-inflammatory drugs are supporting therapies and may include paracetamol, any non-steroidal anti-inflammatory drugs.

Preferably the therapeutic agent is nitisinone.

The compositions of the invention may be combined to any therapeutic intervention known to be effective for the treatment and/or prevention of AKU and HT1, including low protein diet, physiotherapy, joints replacement.

The symbol "-" indicates a single bond, "=" indicates a double bond, "---" indicates the presence or absence of a bond. In the structures drawn and/or described, unless specified otherwise, it is assumed that all the carbon atoms are substituted with a number of hydrogen atoms such as to satisfy the valence of four.

The term "halogen" or "halo" refers to fluorine, chlorine, bromine, or iodine.

The term "alkyl" or "alkyl group" refers to a linear or branched hydrocarbon radical chain. Appropriate examples of said alkyl include but are not limited to methyl, ethyl, *n*-propyl, isopropyl, butyl, *sec*-butyl, *tert*-butyl, pentyl, isopentyl, *tert*-pentyl, hexyl, heptyl, octyl, nonyl, decyl, hexadecyl, eicosanoyl etc. "Substituted alkyl group" means that each hydrogen atom on each carbon atom could be independently replaced by one or more substituents, appropriate examples of substituent include but are not limited to F, Cl, Br, I, CF₃, CN, NH₂, O-C₁₋₆ alkyl, C₁₋₆ alkyl, OH, S-C₁₋₆ alkyl, COC₁₋₆ alkyl, OCOC₁₋₆ alkyl, CO₂C₁₋₆ alkyl, NH-C₁₋₆ alkyl, NHCOC₁₋₆ alkyl, NHCOOC₁₋₆ alkyl, NHCONHC₁₋₆ alkyl.

Preferably the alkyl group is a "C₁₋₁₀ alkyl". "C₁₋₁₀ alkyl" refers to a linear or branched, saturated or unsaturated radical hydrocarbon chain having from one to ten carbon atoms.

The term "haloalkyl" group is an alkyl group as defined above, substituted with one or more halogen atoms, for example one, two, three, four or five halogen atoms; preferably a linear or branched C₁-C₁₀ haloalkyl group, more preferably a Ci-Cs haloalkyl group, more preferably a linear or branched C₁-C₆ haloalkyl group even more preferably a linear or branched C₁-C₄ haloalkyl group, more preferably a C₁-C₂ haloalkyl group, being in particular CF₃, CHF₂, CH₂F. The term "aryl" or "aryl group" represents a mono-substituted, bi- or mono- carbocyclic ring consisting of 5 to 14 atoms, where the mono-cyclic ring is always aromatic and where at least one of the two rings of the bi-cyclic system is aromatic. Appropriate examples of such aryl group include but are not limited to phenyl, naphthyl, and indanyl.

"Substituted aryl" or "substituted aryl groups" means that the hydrogen atom on each carbon atom may be independently replaced by one or more substituents, appropriate examples of substituents include but are not limited to F, Cl, Br, I, CF₃, CN, O-C₁₋₆ alkyl, C₁₋₆ alkyl, OH, S-C₁₋₆ alkyl, COC₁₋₆ alkyl, OCOCC₁₋₆ alkyl, CO₂C₁₋₆ alkyl, NH-C₁₋₆ alkyl, NHCOC₁₋₆ alkyl, NHCOOC₁₋₆ alkyl, NHCONHC₁₋₆ alkyl.

The term "arylalkyl" represents any alkyl group, as defined above, wherein one or more hydrogen atoms are substituted with aryl groups, wherein the aryl is as above defined. Appropriate examples of arylalkyls are represented by benzyl, phenethyl, phenylvinyl, phenylallyl, etc. "Substituted arylalkyl group" means that each hydrogen atom on each carbon atom may be independently replaced by one or more substituents, appropriate examples of substituents include but are not limited to F, Cl, Br, I, CF₃, CN, O-C₁₋₆ alkyl, C₁₋₆ alkyl, OH, S-C₁₋₆ alkyl, COC₁₋₆ alkyl, OCOCC₁₋₆ alkyl, CO₂C₁₋₆ alkyl, NH-C₁₋₆ alkyl, NHCOC₁₋₆ alkyl, NHCOOC₁₋₆ alkyl, NHCONHC₁₋₆ alkyl.

The term "cycloalkyl" or "cycloalkyl group" refers to a monocyclic or polycyclic hydrocarbon ring system having at least three carbon atoms, preferably from three to eight carbon atoms. The cycloalkyl may be completely saturated or partially saturated, but may not contain aromatic rings. The cycloalkyl also includes systems of fused, bridged and spiro rings. Appropriate examples of cycloalkyl groups include cyclopropyl, cyclobutyl, cyclopentyl, cyclohexyl, etc. The term "heteroaryl" or "heteroaryl group" refers to a mono-substituted radical consisting of monocyclic, fused bicyclic or fused tricyclic rings containing from 5 to 14 atoms of which one or more atoms is independently selected from -O-, -S-, -S(O)ₙ- (with n equal to 0, 1, or 2), -N-, - N(R^{x})-, and the remaining ring atoms are carbon atoms, wherein the mono-cyclic ring is always aromatic and wherein at least one of the two fused rings of the bi-cyclic or tri-cyclic system is aromatic. One or two carbon atoms of each non-aromatic ring comprised in the bi-cyclic or tri-cyclic system may be replaced by a -C(=O)-, -C(=S)-, or -C(=NH)- group. R^{x} is H, alkyl, OH or alkoxy. The fused bi-cyclic systems include bridged cyclic systems. Appropriate examples of heteroaryl groups include but are not limited to pyrrole, furan, thiophene, oxazole, isoxazole, 3-methylisoxazole, pyrazole, imidazole, oxadiazole, thiadiazole, triazole, tetrazole, pyridine, pyridazine, pyrimidine, pyrazine, quinoline, isoquinoline, indole, benzofuran, benzothiophene, benzimidazole.

The term "alkoxyl" or "alkoxy" refers to an -O-alkyl group wherein "alkyl" is as defined above. Appropriate examples of alkoxy groups include methoxy, ethoxy, propoxy, butoxy, isopropoxy, isobutoxy, *sec*-butoxy, etc.

The term "carbonyl" refers to a C=O group.

In the context of the invention the salts of the compounds of the present invention are also included. On account of their potential use in medicine, the salts of the compounds of formula I and II are preferably pharmaceutically acceptable. Pharmaceutically acceptable salts comprise the conventional non-toxic salts obtained by salification of a compound of formula I and II with inorganic acids (e.g. hydrochloric, hydrobromic, sulphuric or phosphoric acid), or with organic acids (e.g. acetic, propionic, succinic, benzoic, sulfanilic, 2-acetoxy-benzoic, cinnamic, mandelic, salicylic, glycolic, lactic, oxalic, malic, maleic, malonic, fumaric, tartaric, citric, *p-*toluenesulfonic, methanesulfonic, ethanesulfonic, or naphthalenesulfonic acid). For a review of suitable pharmaceutical salts see [24]. Other salts which are not pharmaceutically acceptable, such as trifluoroacetate salt, can be useful in the preparation of compounds of the present invention and these form a further aspect of the invention. The invention includes within its scope all possible stoichiometric and non-stoichiometric forms of the salts of the compounds of formula I and II.

Furthermore, the compounds of formula I and II can exist in non-solvated forms as well as in forms solvated with pharmaceutically acceptable solvents such as water, EtOH and the like. Some compounds of formula I can exist in stereoisomeric forms (e.g. they may contain one or more asymmetric carbon atoms). The individual stereoisomers (enantiomers and diastereoisomers) and mixtures of these are included in the scope of the present invention. The present invention also covers the individual isomers of the compounds represented by formula I or II as mixtures with isomers in which one or more chiral centres are inverted. Similarly, it is understood that the compounds of formula I and II can exist in tautomeric forms other than those shown in the formula and these are included in the scope of the present invention.

The invention also includes all the suitable isotopic variations of a compound of the invention. An isotopic variation of a compound of the invention is defined as one in which at least one atom is replaced by an atom having the same atomic number but a different atomic mass from the atomic mass usually found in nature. Examples of isotopes which may be incorporated in the compounds of the invention include isotopes such as ²H, ³H, ¹³C, ¹⁴C, ¹⁵N, ¹⁷O, ¹⁸O , ³¹P, ³²P, ³⁵S, ¹⁸F and ³⁶Cl, respectively. Certain isotopic variations of the invention, for example those in which a radioactive isotope such as ³H or ¹⁴C is incorporated, are useful in studies on tissue distribution of the medicament and/or substrate. Furthermore, substitution with isotopes such as deuterium ²H, may provide certain therapeutic advantages resulting from a greater metabolic stability. The isotopic variations of the compounds of the invention can generally be prepared by conventional procedures as well as with the methods illustrated or by the preparations described in the examples below using appropriate isotopic variations of suitable reagents. The invention further comprises pharmaceutical compositions containing at least one compound of the present invention or a pharmaceutically acceptable salt or a solvate thereof and one or more pharmaceutically acceptable carriers, excipients and/or diluents.

The pharmaceutical compositions can be chosen according to the needs of treatment. Such compositions are prepared by mixing and are suitably adapted to oral or parenteral administration, and as such can be administered in the form of tablets, capsules, oral preparations, powders, granules, pills, or injectable or infusible liquid solutions, suspensions, suppositories, preparation by inhalation.

Tablets and capsules for oral administration are usually presented in unit dosage form and contain conventional excipients such as ligands, fillers (including cellulose, mannitol, lactose), diluents, tablet agents, lubricants (including magnesium stearate), detergents, disintegrants (for example polyvinylpyrrolidone and starch derivatives such as sodium starch glycolate), colorants, flavourings and wetting agents (for example sodium lauryl sulphate).

The solid oral compositions may be prepared by conventional methods of mixing, filling or tabletting. The mixing operation can be repeated to distribute the active substance in all the compositions containing large quantities of fillers. Such operations are conventional.

The oral liquid preparations may be in the form of, for example, aqueous or oily suspensions, solutions, emulsions, syrups or elixirs, or may be presented as a dry product for reconstitution with water or with a suitable vehicle before use. Such liquid preparations may contain conventional additives such as suspending agents, for example sorbitol, syrup, methyl cellulose, gelatine, hydroxyethyl cellulose, carboxymethyl cellulose, aluminium stearate gel or hydrogenated edible fats; emulsifying agents such as lecithin, sorbitan monooleate, or acacia; non-aqueous vehicles (which may include edible oils) such as almond oil, fractionated coconut oil, oily esters such as glycerine esters, propylene glycol, or ethyl alcohol; preservatives such as methyl or propyl p-hydroxybenzoate or sorbic acid, and if desired, conventional flavourings or colourants. Oral formulations also include conventional slow-release formulations such as gastro-resistant coated tablets or granules.

The pharmaceutical preparation for administration via inhalation can be provided by an insufflator or by a pressurized nebulizer.

For parenteral administration, the unit dosage of fluid can be prepared comprising the compound and a sterile vehicle. The compound may be suspended or dissolved, depending on the vehicle and concentration. Parenteral solutions are normally prepared by dissolving the compound in a vehicle, sterilizing the latter by filtration, filling suitable vials and sealing. Advantageously, adjuvants such as local anaesthetics, preservatives and buffering agents can be dissolved in the vehicle. To increase stability, the composition can be frozen after having filled the vials and removed water under vacuum. Parenteral suspensions are prepared in substantially the same manner, except that the compound may be suspended in the vehicle instead of being dissolved and sterilized by exposure to ethylene oxide before suspending in the sterile vehicle. Advantageously, a surfactant or a wetting agent may be included in the composition to facilitate uniform distribution of the compound of the invention.

For oral or sublingual administration the compositions may be tablets, lozenges, or gel.

The compounds may be pharmaceutically formulated as suppositories or retention enemas, e.g. suppositories containing conventional bases such as cocoa butter, polyethylene glycol, or other glycerides, for rectal administration.

Another route of administration of the compounds of the invention regards topical treatment. The topical formulations may contain for example ointments, creams, lotions, gels, solutions, pastes and/or can contain liposomes, micelles and/or microspheres. Examples of ointments include oleaginous ointments such as vegetable oils, animal fats, semi-solid hydrocarbons, emulsifiable ointments such as hydroxystearic sulphate, anhydrous lanolin, hydrophilic petrolatum, cetyl alcohol, glycerol monostearate, stearic acid, water soluble ointments containing polyethylene glycols of various molecular weights. The creams, as known to persons skilled in formulation, are viscous liquids or semi-solid emulsions, and contain an oily phase, an emulsifier and an aqueous phase. The oily phase generally contains petrolatum and an alcohol such as cetyl or stearyl alcohol. The formulations suitable for topical ocular administration also include eye drops, in which the active ingredient is dissolved or suspended in a suitable vehicle, in particular in an aqueous solvent for the active ingredient.

A further method of administration of the compounds of the invention regards transdermal vehiculation. Typical transdermal formulations comprise conventional aqueous and non-aqueous vectors, such as creams, oils, lotions or pastes or may be in the form of membranes or medicated patches. A reference for the formulations is Remington's book [25].

The compounds of the present invention can be used in the treatment and/or prevention of the conditions referred to herein as a single therapy or in combination with other therapeutic agents, or by separate administrations, or including the two or more active substances in the same pharmaceutical formulation. The compounds can be administered simultaneously or sequentially.

The other therapeutic agents may be compounds currently on the market. Non-exhaustive examples of suitable additional agents include in particular compound 1 or its derivatives.

The combination can be administered as a separate composition (simultaneous, sequential) of the individual components of the treatment or as a single dosage form containing both agents. When the compounds of the present invention are combined with other active substances, the active substances may be formulated separately in the form of single ingredient preparations of one of the forms described above, and then given as combined preparations, administered at the same time or different times, or may be formulated together in preparations with two or more ingredients.

The compounds of general formula I and II may be administered to a patient in a total daily dose of, for example, 0.001 to 1000 mg/kg of body weight per day. The compositions of the dosage units may contain amounts of sub-multiples thereof to make up the daily dose. The compound can also be administered weekly or every other day. The determination of optimum dosages for a specific patient is well known to those skilled in the art. As is common practice, the compositions are normally accompanied by instructions for use in the treatment in question written or printed.

The present invention will now be illustrated by means of non-limiting examples with reference to the following figures.

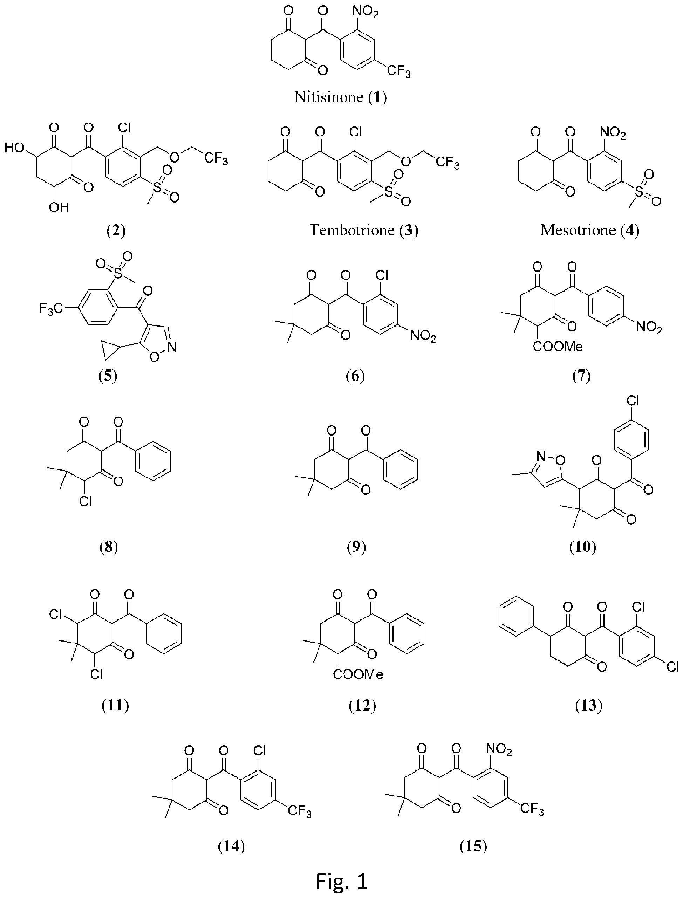

**Figure 1****.** Chemical structure of analysed compounds. **(1)** 2-(2-nitro-4-(trifluoromethyl)benzoyl)cyclohexane-1,3-dione, **(2)** 2-[2-chloro-4-methylsulfonyl-3-(2,2,2-trifluoroethoxymethyl)benzoyl]-4,6-dihydroxy-cyclohexane-1,3-dione, **(3)** 2-[2-chloro-4-methylsulfonyl-3-(2,2,2-trifluoroethoxymethyl)benzoyl]cyclohexane-1,3-dione, **(4)** 2-(2-nitrobenzoyl-4-methylsulfonyl)cyclohexane-1,3-dione, **(5)** (5-cyclopropylisoxazol-4-yl)(4-trifluoromethyl)-2-(methylsulfonyl)phenyl)methanone, **(6)** 2-(2-chloro-4-nitro-benzoyl)-5,5-dimethyl-cyclohexane-1,3-dione, **(7)** 2,2-dimethyl-5-(4-nitrobenzoyl)-4,6-dioxycyclohexane methyl carboxylate, **(8)** 2-benzoyl-4-chloro-5,5-dimethyl-cyclohexane-1,3-dione, **(9)** 2-benzoyl-5,5-dimethyl-cyclohexane-1,3-dione, **(10)** 2-(4-chlorobenzoyl)-5,5-dimethyl-4-(3-methylisoxazol-5-yl)-cyclohexane-1,3-dione, **(11)** 2-benzoyl-4,6-dichloro-5,5-dimethylcyclohexane-1,3-dione, **(12)** 5-benzoyl-2,2-dimethyl-4,6-dioxacyclohexane methyl carboxylate, **(13)** 2-(2,4-dichlorobenzoyl)-4-phenyl-cyclohexane-1,3-dione, **(14)** 2-(2-chloro-4-trifluoromethylbenzoyl)-5,5-dimethyl-cyclohexane-1,3-dione, **(15)** 2-(2-Nitro-4-trifluoromethylbenzoyl)-5,5-dimethyl-cyclohexane-1,3-dione.

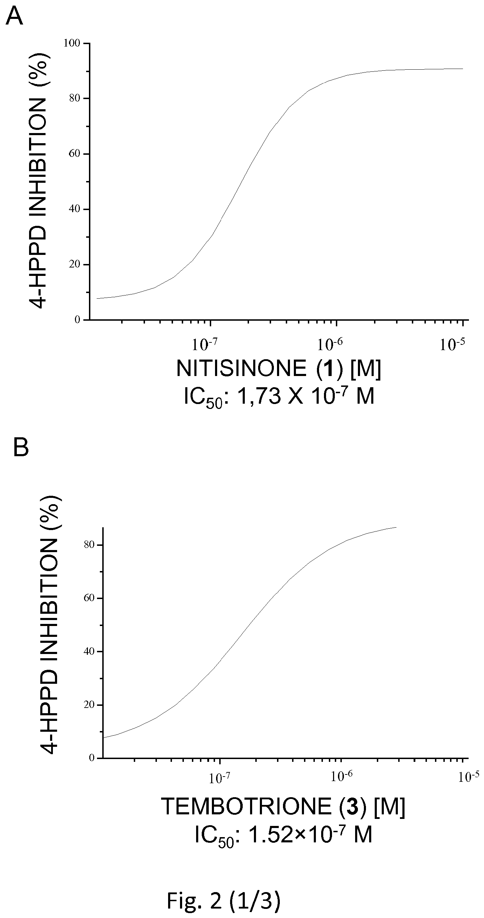

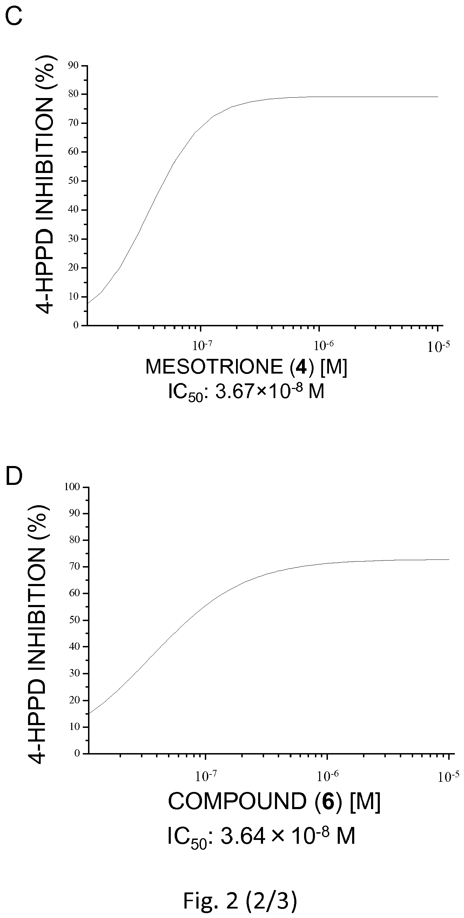

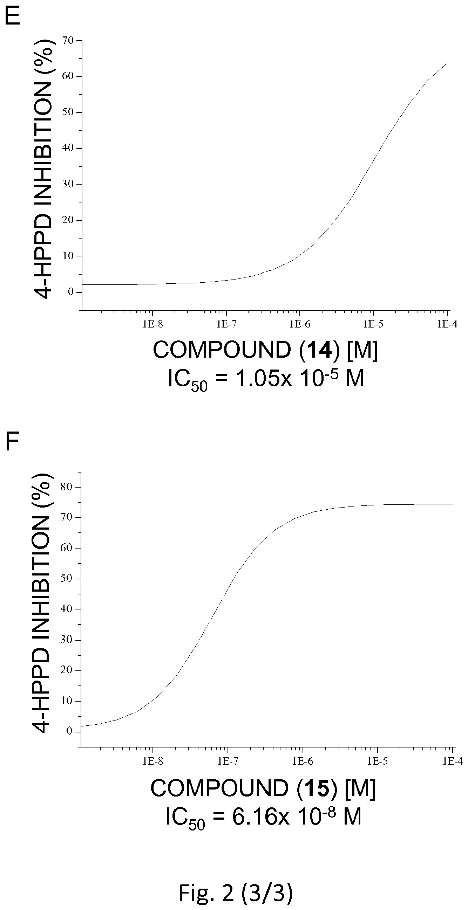

**Figure 2****.** Determination of inhibition of the catalytic activity of the enzyme 4-HPPD by different compounds of the invention: A, compound **1;** B, compound **3;** C, compound **4;** D, compound **6;** E, compound **14;** F, compound **15** and relative IC₅₀.

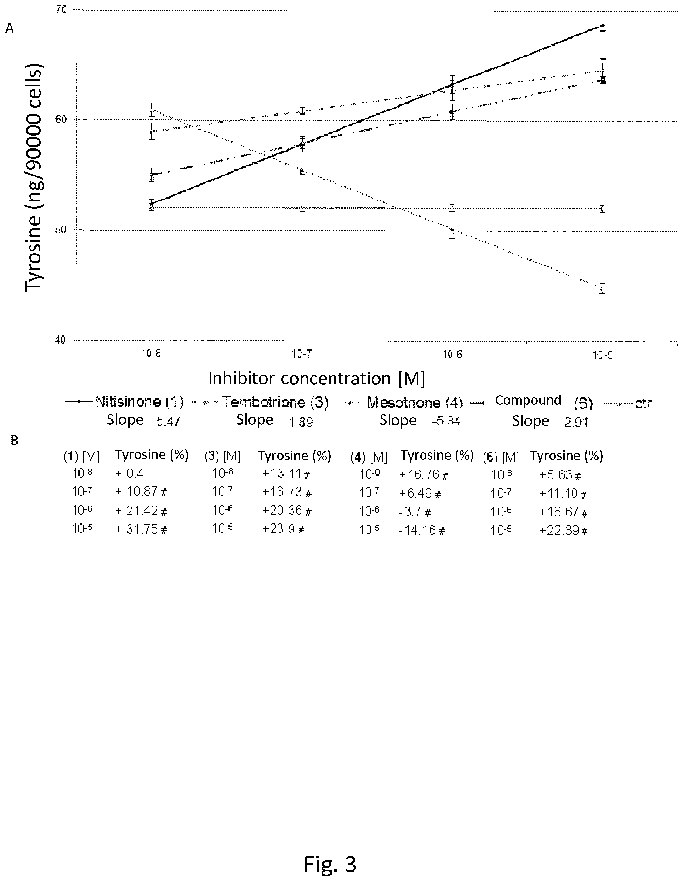

**Figure 3****.** A. Levels of tyrosine in human fibroblasts (HFb) treated for 72 hours with compounds **1, 3, 4, 6** of the invention and treated with the DMSO vehicle (control). The intracellular tyrosine ng in 9×10⁴ cells is plotted for each concentration of tested compound; the slope of each dose-response curve is shown in the figure. Data are expressed as mean value ± SD of three experiments. B. Percentage of intracellular tyrosine accumulation in HFb treated with the compounds **1, 3, 4, 6** at the tested concentrations, compared to the control (HFb treated with the vehicle). (+) indicates an increase compared to the control (HFb treated with the vehicle), while (-) indicates a reduction compared to the control. # *p*<0.05 compared to the value in untreated HFb.

### DETAILED DESCRIPTION OF THE INVENTION

### Materials and methods

### Compounds

Compounds **1-13** of Figure 1 were supplied by Siena Biotech SpA, while compounds **14** and **15** were synthesized as described below. The purity of the compounds was ≥95% as determined by HPLC, and they were solubilised with DMSO.

Preparation of 2-(2-nitro-4-trifluoromethylbenzoyl)-5,5-dimethyl-cyclohexane-1,3-dione (compound **15).**

2-Nitro-(4-trifluoromethyl)-benzoic acid (200 mg, 0.85 mmol) was dissolved in thionyl chloride (4 mL) and refluxed under nitrogen for 2 h. After coevaporation with toluene (2 × 5 mL) and dichlorometane (2 × 5 mL) under reduced pressure, a yellow oil was obtained and directly dissolved in 6 mL of dry dichlorometane. 5,5-Dimethyl-1,3-cyclohexanedione (dimedone, 239 mg, 1.7 mmol) and triethylamine (TEA, 383 µL, 3.25 mmol) were added under nitrogen. The reaction mixture was stirred at room temperature monitoring it by thin-layer chromatography (petroleum ether and ethyl acetate 1:1). After 2 h, the reaction was washed with 1 M hydrochloric acid (2 × 5 mL). The organic phases was dried over dry sodium sulphate and the solvent evaporated under reduced pressure after filtration. The crude was purified by column chromatography using dichlorometane and methanol (9:1). A pale yellow solid was obtained in 10% yield.

¹H NMR: (400 MHz, CDCl₃) δ 16.20 (s, 1H), 8.44 (s, 1H), 7.92 (d, *J* = 7.9 Hz, 1H), 7.36 (d, *J* = 7.9 Hz, 1H), 2.64 (s, 2H), 2.21 (s, 2H), 1.08 (s, 6H) ppm. ¹³C NMR: (101 MHz, CDCl₃) δ 195.84, 194.55, 194.02, 139.56, 130.82, 130.78, 127.82, 121.18, 121.14, 111.84, 51.18, 45.26, 31.29, 31.27, 29.68, 28.13 ppm. ESI MS: 380 [M+Na]⁺.

Preparation of 2-(2-chloro-4-trifluoromethylbenzoyl)-5,5-dimethyl-cyclohexane-1,3-dione (compound **14).**

2-Chloro-(4-trifluoromethyl)-benzoic acid (200 mg, 0.89 mmol) was dissolved in thionyl chloride (4 mL) and refluxed under nitrogen for 2 h. After coevaporation with toluene (2 × 5 mL) and dichlorometane (2 × 5 mL) under reduced pressure, a yellow oil was obtained and directly dissolved in 6 mL of dry dichlorometane. 5,5-Dimethyl-1,3-cyclohexanedione (dimedone, 125 mg, 0.89 mmol) and triethylamine (TEA, 161 µL, 1.16 mmol) were added under nitrogen. The reaction mixture was stirred at room temperature monitoring it by thin-layer chromatography (petroleum ether and ethyl acetate 1:1). After 3 h, the reaction was washed with 1 M hydrochloric acid (2 × 5 mL). The organic phases was dried over dry sodium sulphate and the solvent evaporated under reduced pressure after filtration. The crude was purified by chromatography column using dichlorometane and methanol (9:1). A pale yellow oil was obtained in 11% yield.

¹H NMR: (400MHz, CDCl₃): δ 7.99 (d, 1H, J=8 Hz), 7.75 (s, 1H), 7.60 (d, 1H, J=8 Hz), 6.04 (s, 1H), 2.54 (s, 2H), 2.30 (s, 2H), 1.13 (s, 6H) ppm. ¹³C NMR: (100 MHz, CDCl₃): δ 199.17, 167.80, 161.40, 135.69, 135.28, 132.54, 131.94, 128.67, 128.63, 124.13, 123.92, 117.42, 51.05, 42.27, 33.52, 28.39 ppm. ESI MS: 369 [M+Na]⁺.

### Determination of 4-HPPD activity

4-HPPD activity was evaluated in the liver cytosol of a male Wistar rat by monitoring oxygen consumption through the use of the SDR SensorDish^{®} Reader (Presens, Precision Sensing GmbH, Regensburg, Germany). Briefly, a special test tube with an oxygen sensor placed on the bottom was filled with 0.2 M sodium phosphate buffer with pH 7.4, 1.7 mM ascorbic acid (used as a cofactor) and 10.92 mg of rat enzyme preparation (4-HPPD source). The mixture was equilibrated at 37°C for 5 min. and the reaction was started by the addition of substrate (0.2 M of HPP, Sigma-Aldrich cod. SAE0003). Incubation was performed in the presence of compound **(1),** or the other inhibitors at different concentrations (10⁻⁸, 10⁻⁷, 10⁻⁶ and 10⁻⁵ M). For each test compound, non-interference with the assay components was confirmed by carrying out a negative control with an enzyme-free solution. The percentage of inhibition of enzymatic activity was calculated as the ratio of the slopes (decrease of the concentration of O₂ (µmol/L) *per* min.) of the samples containing the inhibitor *versus* the untreated controls corrected with the negative control values. The IC₅₀ values were calculated by means of regression analysis in the range of tested concentrations by means of the Origin^{®} - Data Analysis and Graphing software using a sigmoid type interpolating curve and plotting on the x-axis the concentrations of the compounds and on the y-axis oxygen consumption values expressed as a percentage of 4-HPPD inhibition, the reduction of oxygen being correlated with inhibition of the enzyme.

### Assessment of cytotoxicity

12×10⁴ primary human fibroblasts (ATCC, cat PCS-201-012) were treated with compounds at various concentrations (10⁻⁷ M, 10⁻⁶ M, 10⁻⁵ M, 10⁻⁴ M, 10⁻³ M) for 24 and 144 h. HFb were cultured as described in [21]. Cellular viability was quantified by the MTT assay (Sigma-Aldrich cod. M5655) as described in [22]. Controls were performed treating the cells with vehicle (DMSO) alone. Calculation of LD₅₀ was performed by means of the Origin^{®} - Data Analysis and Graphing software using a sigmoid type interpolating curve.

### Tyrosine dosage

Intracellular tyrosine was calculated in HFb (9×10⁴ cells/well) treated for 72 h with the compounds under assessment at various concentrations (10⁻⁸, 10⁻⁷, 10⁻⁶ and 10⁻⁵ M); controls were performed using the carrier at the concentration corresponding to the highest dose of the compound under assessment. At the end of treatment, the cells were recovered by scraping in the presence of norvaline (10⁻³ M, used as internal standard, Sigma-Aldrich cod. N7627) and cold methanol (40 µL, Sigma-Aldrich cod. 34860), and subsequently lysed by cycles of freezing/thawing in a bath of dry ice/nitrogen. The lysate was centrifuged (6000g for 5 min) and the supernatant was then recovered, evaporated and re-suspended with 100 µL of 0.05% formic acid. Each sample was analysed by LC-MS-MS. In particular, the analysis was performed with a chromatographic instrument manufactured by Varian (Varian Inc.) run by the software Varian MS Workstation System Control Vers. 6.9 and consisting of 2 pumps (212-LC) connected to a triple quadrupole detector Varian (Mod. 320-LC) fitted with ESI ionization source (Varian Inc.). Subsequently, the concentration of each compound was correlated with the amount of intracellular tyrosine contained in 9×10⁴ cells and the resulting dose-response curve was obtained using the Origin^{®} - Data Analysis and Graphing software by means of a linear type interpolating curve.

Percentage of tyrosine accumulation in HFb treated with compounds **1, 3, 4,** and **6** at various concentrations was calculated assuming the value 100% to the control (DMSO-treated HFb) and then the relative % fold change was obtained.

### Statistical analysis

All the experiments were conducted in triplicate; the data were presented as mean values with standard error. The Student's t-test was used when necessary; differences having a value of at least *p*≤0.05 were considered significant.

### Results

In the present invention, compound **1** and twelve commercial and two novel 4-HPPD inhibitors (Figure 1) were subjected to a pharmacodynamic and toxicology study in order to assess IC₅₀, LD₅₀ and the possible accumulation of tyrosine. 4-HPPD inhibition was determined in the cytosolic extract of rat liver (source of the enzyme), by evaluation of enzymatic consumption of oxygen using the SDR SensorDish ^{®} Reader, and was expressed as an IC₅₀ value.

LD₅₀ was evaluated *in vitro* using the MTT cell viability assay for the first time on human cells, in particular on primary human fibroblasts (HFb). The expression of homogentisate 1,2-dioxygenase (HGD) in said HFb was demonstrated by Western Blot analysis (data not shown). Intracellular tyrosine was measured in HFb by LC-MS-MS analysis.

IC₅₀ and LD₅₀ values for the compounds of the invention are shown in Table 1.

**Table 1. IC₅₀ and LD₅₀ values obtained for compound (1) and for the compounds of the invention.**

| 4-HPPD inhibitor | LD₅₀ [a] | | IC₅₀ [a] |
|---|---|---|---|
| | 24 hours | 144 hours | |
| **1,** Nitisinone | >1 mM [c] | ~1 mM | 173 ± 1 nM |
| **2** | 0.48 ± 0.04mM | 0.48 ± 0.03mM | 227 ± 9 nM |
| **3,** tembotrione [b] | >1 mM [c] | >1 mM [c] | 152 ± 6 nM |
| **4,** mesotrione, [b] | 1.1 ± 0.1 mM | >1 mM [c] | 36.7 ± 9.7 nM |
| **5** | >1 mM [c] | 0.11 ± 0.07 mM | 399 ± 19 nM |
| **6** [b] | >1 mM [c] | >1 mM [c] | 36.4 ± 9.3 nM |
| **7** | 0.63 ± 0.12 mM | >1 mM [c] | no inhibition |
| **8** | >1 mM [c] | >1 mM [c] | no inhibition |
| **9** | >1 mM [c] | >1 mM [c] | no inhibition |
| **10** | >1 mM [c] | 0.82 ± 0.20 mM | 59.9 ± 1.8 µM |
| **11** | >1 mM [c] | 0.29 ± 0.08 mM | 192 ± 2 nM |
| **12** | >1 mM [c] | >1 mM [c] | no inhibition |
| **13** | >1 mM [c] | 0.36 ± 0.15 mM | 279 ± 6 nM |
| **14** | >10 µM | | 10.5 ± 3.7 µM |
| **15** | > 10 µM | | 61.6 ± 7.4 nM |

| | | | |
|---|---|---|---|
| [a] The values are presented as the mean value of n = 3 experiments performed in triplicate. [b] Compounds with more favourable ICso and LD₅₀ values than compound **(1).** [c] The LD₅₀ value of >1 mM derives from the fact that more than 50% of HFb were still viable when the concentration of the compound was >1 mM and therefore the actual value of LD₅₀ is greater than 1 mM. | | | |

On the basis of the IC₅₀ and LD₅₀ values reported in Table 1, compounds **3, 4** and **6** were selected for subsequent experiments. Such compounds are potent 4-HPPD inhibitors (low IC₅₀ values) and display low cytotoxicity (high LD₅₀ values). The compounds are improved in respect to compound **1.**

The profile of each compound will be discussed separately.

### Compound 1

As shown in Table 1 and Figure 2A, compound **1** showed a sub-micromolar 4-HPPD inhibitory activity (1.73×10⁻⁷ M). With regards to cytotoxicity, it was not possible to calculate the LD₅₀ at 24 h because more than 50% of the HFb were still viable at that time point. Therefore, the LD₅₀ at 24 h was indicated as >10⁻³ M in Table 1. On the other hand, the LD₅₀ at 144 h was 10⁻³ M. The fact that the long-term value of LD₅₀ (144 h) is lower than the short-term value (24 h) supports the hypothesis that the toxicity of compound 1 is linked to its mechanism of action (i.e, irreversible inhibition of 4-HPPD with consequent hyper-tyrosinemia *in vivo* after prolonged administration) [10, 14, 15, 18].

These data were further confirmed by the results obtained in the intracellular tyrosine assay on HFb as shown in Figure 3. This is the first experimental evidence of a massive intracellular tyrosine accumulation in human cell cultures after treatment with compound **1.** In fact, as shown in Figure 3B, when HFb were treated with compound **1** at concentrations of 10⁻⁶ M and of 10⁻⁵ M, intracellular tyrosine increased by about 21% and 32%, respectively, compared to untreated HFb. These two concentrations correspond to the plasma levels of free compound **1** observed in AKU patients [15, 17]. Long-term treatment of AKU patients with compound **1** induced a 10- to 14-fold increase in tyrosine levels when compared to untreated AKU patients [4, 15, 17]. In the present cellular model tyrosine accumulation of 1.1- to 1.3-fold change relative to control is observed after 72 h.In addition, the present results showed that tyrosine accumulation following treatment with compound **1** is dose-dependent, as shown in Figure 3A.

### Compound 3

Compound **3** (Tembotrione) shows a very interesting activity profile, with an IC₅₀ of 1.52×10⁻⁷ M and an LD₅₀ greater than 10⁻³ M, which is higher than that of compound **1** (Table 1 and Figure 2B). This means that the compound is able to inhibit 4-HPPD analogously to compound **1** but with a better cytotoxicity profile. These results are consistent with and corroborated by the data obtained in the intracellular tyrosine assay. In fact, by comparing the slopes of the respective curves shown in Figure 3A, the increase in intracellular tyrosine levels induced by the treatment with compound **3** is lower (slope of 1.89) than that induced by the treatment with compound **1** (slope of 5.47). Consequently, concerning the relationship between cytotoxicity and mechanism of action, compound **3** is presumably safer than compound **1** from a toxicological point of view and at the same time equally active as 4-HPPD inhibitor.

### Compound 4

Compound **4** (Mesotrione) also shows a very interesting profile both in terms of in vitro enzyme inhibition and cytotoxicity. In fact, as shown in Table 1 and Figure 2C, the IC₅₀ (3.67×10⁻⁸ M) is four-time lower than that of compound **1,** indicating an increased 4-HPPD inhibition potency. In addition, as shown from Table 1, the LD₅₀ after a 144 h treatment with compound **4** was higher than that of compound **1,** suggesting a lower cytotoxicity of compound **4** particularly in long-term treatment.

As shown in Figure 3, the intracellular tyrosine assay indicated that, at the lowest inhibitor concentration analysed, the intracellular tyrosine levels were about 17% higher than in the control group (*p*<0.01). On the other hand, when concentrations of compound **4** were increased, the intracellular tyrosine levels decrease significantly if compared to the control (p<0.05). In particular, at the highest inhibitor concentration, the tyrosine level was about 14% lower than in the control (*p*<0.01). This may suggest that the compound induces a massive blockage of 4-HPPD (confirmed by low IC₅₀). In turn, such blockage could activate alternative (usually inactive) pathways of tyrosine catabolism [23]. The hypothesis of a massive 4-HPPD block is supported also by the results of the cytotoxicity assay. The fact that LD₅₀ of compound **4** is lower after 24 h (in the short term) than at 144 h (in the long term) can be explained by a potential initial toxicity due to a massive initial tyrosine accumulation. The trend in tyrosine levels seen after administration of compound **4** as shown in Figure 3A is certainly advantageous for a potential treatment of AKU and HT1. In fact, the accumulation of HGA, succinyl acetoacetate, and succinylacetone can be reduced without any increase in the plasma tyrosine concentration.

### Compound 6

The biological results obtained for compound **6** suggest that it may be the most promising compound in terms of 4-HPPD inhibition, with low cytotoxicity and tyrosine accumulation.

Its IC₅₀ value of ~36.4 nm (Table 1 and Figure 2D) makes this compound effective in vitro at very low concentrations. The residual activity of the enzyme is nearly 30%. The compound is also safe as the viability of HFb after 144 h of treatment with a concentration 1 mM is nearly 75%. This concentration is 500-fold higher than plasma levels of compound **1** observed in AKU patients in a clinical trial [4, 15, 17], thereby underscoring the safety of compound **6.** Moreover, at the highest concentration tested, the inventors observed an intracellular tyrosine accumulation of about 7% lower than that found for compound **1** (p<0.01), after treatment for three days. Consequently, although the comparison between in vitro and in vivo models must be performed with caution, the data obtained for compound **6** suggest that this compound allows for an easy and safe modulation of 4-HPPD catalytic activity in vivo. In fact, in a therapeutic context such as that of AKU and HT1, the ideal characteristic of a drug is its ability to regulate the enzymes involved in the production of HGA and of succinyl acetoacetate and succinylacetone without causing harmful tyrosine accumulation.

It should also be noted that for both compounds **4** and **6** the IC₅₀ and LD₅₀ values were very far apart, indicating that the compounds are capable of producing the desired pharmacological effect without generating side effects associated with their mechanism of action.

### Compounds 14 and 15

Two further compounds were tested: 2-(2-chloro-4-trifluoromethylbenzoyl)-5,5-dimethylcyclohexane-1,3-dione, compound **14,** and 2-(2-nitro-4-trifluoromethylbenzoyl)-5,5-dimethylcyclohexane-1,3-dione, compound **15:**

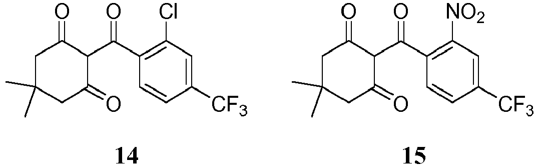

Compound **14** shows an IC₅₀ = 10.5 ± 3.7 µM and LD₅₀ >10 µM (24 h), while compound **15** shows an IC₅₀ = 61.6 ± 7.4 nM and LD₅₀ > 10 µM (24 h). Comparing the IC₅₀ values of compounds **14** and **15** with those obtained for the previously listed triketones (Table 1), it was found that compound **15** was a more potent 4-HPPD inhibitor than compounds **1** and **3.** Compound **15** had similar 4-HPPD inhibition activity to compounds **6** and **4.** On the contrary, compound **14** has a lower 4-HPPD inhibition activity when compared to compound **1.** Concerning toxicity, compounds **14** and **15** were not cytotoxic as demonstrated by low LD₅₀ values.

The present invention provides toxicological data for compound **1** in particular its LD₅₀ in human cells after acute (24 hours) and chronic (144 hours) treatment. Moreover, the present invention provides novel 4-HPPD inhibitors, capable of reducing the levels of HGA and succinyl acetoacetate and succinylacetone while minimizing increase in plasma tyrosine. Then the present inhibitors are particularly advantageous for the treatment of AKU and HT1, especially as a lifetime treatment [4, 9, 15, 18].

The present 4-HPPD inhibitors display a more beneficial profile than compound **(1)** in terms of enzyme inhibition (IC₅₀), cytotoxicity (LD₅₀) and tyrosine accumulation. Such compounds may to be used for the treatment of AKU and HT1.

### BIBLIOGRAPHY

[1] D Braconi, et al., Expert Rev. Proteomics 2013, 10(6), 521-35.
[2] D Braconi, et al., Free Radic. Biol. Med. 2015, 88(Pt A), 70-80
[3] M Laschi, et al., J. Cell. Physiol. 2012, 227(9), 3254-7.
[4] LR Ranganath, AM. Milan, et al., Ann. Rheum. Dis. 2016, 75(2), 362-7.
[5] JB Amoux, et al., J. Inherit. Metab. Dis. 2015, 38(5), 791-6.
[6] DL Lee, et al., Weed Sci. 1998, 45, 601-9.
[7] R Beaudegnies, et al., Bioorg. Med. Chem. 2009, 17(12), 4134-52.
[8] PJ McKiernan, MA Preece, A Chakrapani. Arch. Dis. Child. 2015, 100(8), 738-41.
[9] EMEA 2005: European Public Assessment Report for Orfadin Scientific Report. EMA/823169/2009, EMEA/H/C/555.
[10] A Masurel-Paulet, et al., J. Inherit. Metab. Dis. 2008, 31(1), 81-7.
[11]F Bendadi, et al., J. Pediatr. 2014, 164(2), 398-401.
[12] EA Lock, et al., J. Inherit. Metab. Dis. 1998, 5, 498-506.
[13] EA Lock, et al., Toxicology 2000, 144(1-3), 179-87.
[14] EA Lock, et al., Toxicol. Appl. Pharmacol. 2006, 215(1), 9-16.
[15] WJ Introne, et al., Mol. Genet. Metab. 2011, 103(4), 307-14.
[16] C Phomphutkul, et al., N. Engl. J. Med. 2002, 347(26), 2111-21.
[17] P Suwannarat, et al., Metabolism 2005, 54(6), 719-28.
[18] B Olsson, et al., JIMD Rep. 2015, 24, 21-7.
[19]AusPAR: Nitisinone. http://www.tga.gov.au/auspar/auspar-nitisinone.
[20] SmPC for Orfadin capsules. http://www.ema.europa.eu/docs/en_GB/document_library/EPAR_Product_Information/hu man/000555/WC500049195.pdf
[21] S Sestini, et al., Eur. J. Orthod. 2006, 28(6), 567-72.
[22] A Spreafico, et al., FASEB J. 2008, 22(5), 1560-71.
[23] MG Hall, et al., Br. J. Clin. Pharmacol. 2001, 52(2), 169-77.
[24] SM Berge, et al., J. Pharm. Sci. 1977, 66, 1-19; PL Gould, Int. J. Pharm. 1986, 33, 201-217; LD Bighley, et al., Encyclopedia of Pharmaceutical Technology, Marcel Dekker Inc, New York 1996, Volume 13, page 453-497.
[25] Remington "The Science and Practice of Pharmacy", Lippincott Williams & Wilkins, 2000**.**

## Claims

1. A compound of formula I or II for use in the prevention and/or treatment of a disease **characterized by** accumulation of homogentisic acid (HGA) or of succinyl acetoacetate and succinylacetone: 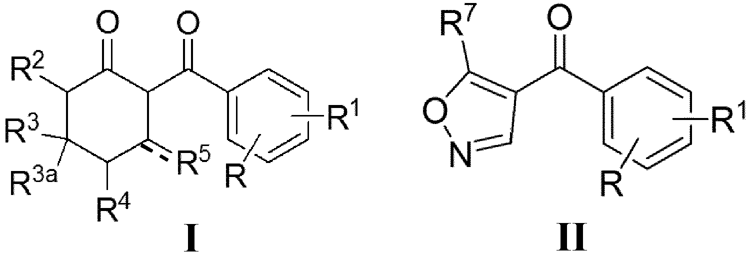 wherein
--- represents the presence or absence of a bond;
- R and R¹ are identical or different and independent of each other, in ortho, meta or para position, and represent -H; -OH; -NH₂; -NO₂; -SO₂CH₃; a halogen atom; an alkyl group;
a haloalkyl group; an alkoxy group -O-alkyl; a cycloalkyl group; a phenyl group or an aryl group;
- R², R³, R^{3a}, R⁴ and R⁵ are identical or different and independent of each other, and represent -H; -OH; -NH₂; -NO₂; -SO₂CH₃; a halogen atom; an alkyl group; a carbonyl group; a cycloalkyl group; an alkoxy group; a phenyl group; an aryl group, or a heteroaryl group; an amide group -C(=O)-NH-R⁶ or an ester group -C(=O)-O-R⁶;
further, R⁵ may be oxo when the bond between R⁵ and the carbon to which R⁵ is attached is a double bond,
further, keeping the above defined substituents for R and R¹, the groups R² and R³ or R^{3a}, R³ or R^{3a} and R⁴, R⁴ and R⁵ may be fused to form, together with the carbon atoms adjacent to the cyclohexanone group on which they are located, a cycle with 5, 6 or 7 atoms condensed to the same cyclohexanone;
- R⁶ represents an alkyl group, a cycloalkyl group, a phenyl group, an aryl group or a heteroaryl group;
- R⁷ represents: -H; -OH; -NH₂; -NO₂; -SO₂CH₃; a halogen atom; an alkyl group; an alkoxy group; a cycloalkyl group; a phenyl group or an aryl group,
wherein the compound of formula I in which R is ortho-NO₂, R¹ is para-CF₃, R² = R³ = R^{3a} = R⁴ and is H and R⁵ is oxo when the bond between R⁵ and the carbon to which R⁵ is attached is a double bond (known as Nitisinone) is not included.

2. The compound of formula I for use according to claim 1 wherein:
- the halogen atom is Cl or Br and/or;
- the alkyl group comprises a carbon chain that contains from 1 to 10 carbon atoms, said chain being linear or branched, saturated or unsaturated, with one or more double bonds and/or one or more triple bonds, said chain being unsubstituted or substituted with one or more heteroatoms such as O, S, or N, or with one or more halogen atoms, or with one or more aryl or heteroaryl groups and/or;
- the haloalkyl group is CF₃, CHF₂, CH₂F and/or;
- the cycloalkyl group comprises from 3 to 8 carbon atoms, preferably the cycloalkyl group is cyclopropyl, cyclopentyl or cyclohexyl and/or;
- the heteroaryl group is pyrrole, furan, thiophene, oxazole, isoxazole, 3-methylisoxazole, pyrazole, imidazole or pyridine.

3. The compound of formula I for use according to claim 1 or 2 wherein:
- R is H, *ortho*-chloro or *ortho*-NO₂;
- R¹ is H, *para-*Cl*, para*-SO₂CH₃, *para*-NO₂, *para*-CF₃;
- R² is H, Cl, OH, phenyl, 3-methlylisoxazol-6-yl;
- R³ e R^{3a} are H or CH₃;
- R⁴ is H, OH, Cl, COOCH₃ and
- R⁵ is H or oxo when the bond between R⁵ and the carbon to which R⁵ is attached is a double bond.

4. The compound of formula I for use according to claim 1 or 2 wherein the compound is selected from the group consisting of:
- 2-[2-Chloro-4-methylsulfonyl-3-(2,2,2-trifluoroetoxyimethyl)benzoyl]-4,6-dihydroxy-cyclohexane-1,3-dione, compound **2:** 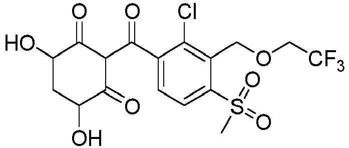
- 2-[2-Chloro-4-methylsulfonyl-3-(2,2,2-trifluoroetoxymethyl)benzoyl]cyclohexane-1,3-dione, known as tembotrione, compound **3:** 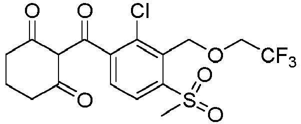
- 2-(4-Methylsulfonyl-2-nitrobenzoyl)-cyclohexane-1,3-dione, known as mesotrione, compound **4:** 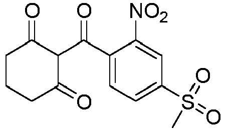
- 2-(2-chloro-4-nitro-benzoyl)-5,5-dimethyl-cyclohexane-1,3-dione, compound **6:** 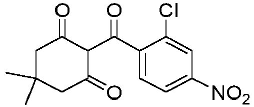
- 2,2-Dimethyl-5-(4-nitrobenzoyl)-4,6-dioxycyclohexane methyl carboxylate, compound **7:** 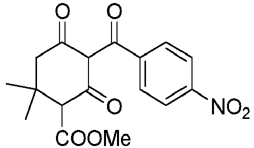
- 2-Benzoyl-4-chloro-5,5-dimethyl-cyclohexane-1,3-dione, compound **8:** 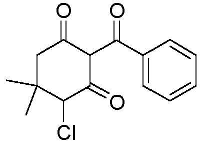
- 2-Benzoyl-5,5-dimethyl-cyclohexane-1,3-dione, compound **9:** 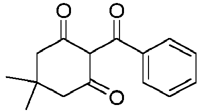
- 2-(4-chlorobenzoyl)-5,5-dimethyl-4-(3-methyl-isoxazol-5-yl)-cyclohexane-1,3-dione, compound **10:** 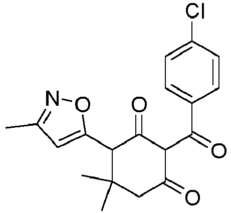
- 2-Benzoyl-4,6-dichloro-5,5-dimethyl-cyclohexane-1,3-dione, compound **11:** 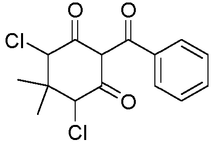
- 5-Benzoyl-2,2-dimethyl-4,6-dioxacyclohexane methyl carboxylate, compound **12:** 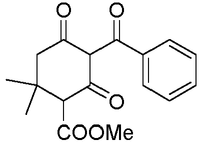
- 2- (2,4-dichlorobenzoyl) -4-phenyl-cyclohexane-1,3-dione, compound **13:** 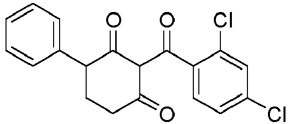
- 2-(2-Chloro-4-trifluoromethylbenzoyl)-5,5-dimethyl-cyclohexane-1,3-dione, compound **14:** 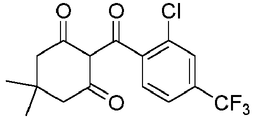
- 2-(2-Nitro-4-trifluoromethylbenzoyl)-5,5-dimethyl-cyclohexane-1,3-dione, compound **15:**

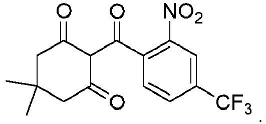

5. The compound of formula I for use according to claim 1 or 4 wherein the compound is selected from the group consisting of:

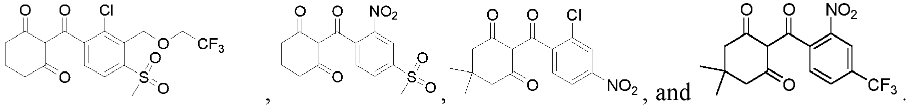

6. The compound of formula I or II for use according to any one of claim 1 to 5 wherein the disease **characterized by** accumulation of HGA is alkaptonuria.

7. The compound of formula I or II for use according to any one of claim 1 to 5 wherein the disease **characterized by** accumulation of succinyl acetoacetate and succinylacetone is tyrosinemia type I.

8. The compound of formula I or II for use according to any one of claim 1 to 7 in combination with a therapeutic intervention.

9. The compound of formula I or II for use according to claim 8 wherein the therapeutic intervention is selected from the group consisting of: low protein diet, administration of 4-HPPD inhibitor, anti-oxidants, pain killers, anti-inflammatory drugs, physiotherapy, joints replacement.

10. The compound of formula I or II for use according to claim 8 or 9 wherein the further therapeutic intervention is nitisinone.

11. A compound of formula I being

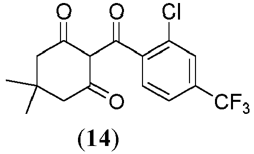

12. The compound according to claim 11 in combination with a therapeutic intervention, preferably the therapeutic intervention is selected from the group consisting of: low protein diet, administration of 4-HPPD inhibitor, anti-oxidants, pain killers, anti-inflammatory drugs, physiotherapy, joints replacement, preferably the further therapeutic intervention is nitisinone.

13. A pharmaceutical composition comprising the compound **(14)** or **(15)** as defined in claim 4 and a pharmaceutically acceptable excipient.

14. A pharmaceutical composition comprising at least one compound of formula I or II as defined in any one of claim 1 to 5 and a pharmaceutically acceptable excipient for use in the prevention and/or treatment of a disease **characterized by** accumulation of HGA or of succinyl acetoacetate and succinylacetone, preferably the pharmaceutical composition further comprises a therapeutic agent, preferably the therapeutic agent is nitisinone.

## Patentansprüche

1. Verbindung der Formel I oder II zur Verwendung bei der Prävention und/oder Behandlung einer Erkrankung, die **gekennzeichnet ist durch** eine Akkumulation von Homogentisinsäure (HGA) oder von Succinylacetoacetat und Succinylaceton: 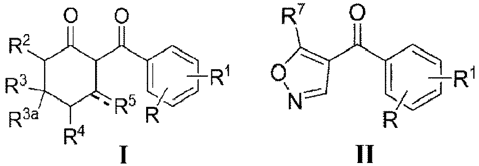 worin
--- das Vorhandensein oder Nichtvorhandensein einer Bindung darstellt;
- R und R' gleich oder verschieden und unabhängig voneinander, in ortho-, meta- oder para-Stellung sind, und für -H; -OH; -NH₂; -NO₂; -SO₂CH₃; ein Halogenatom;
eine Alkylgruppe; eine Halogenalkylgruppe; eine Alkoxygruppe -O-Alkyl; eine Cycloalkylgruppe; eine Phenylgruppe oder eine Arylgruppe stehen;
- R², R³, R^{3a}, R⁴ und R⁵ gleich oder verschieden und unabhängig voneinander sind, und für -H; -OH; -NH₂; - NO₂; -SO₂CH₃; ein Halogenatom; eine Alkylgruppe; eine Carbonylgruppe; eine Cycloalkylgruppe; eine Alkoxygruppe; eine Phenylgruppe; eine Arylgruppe oder eine Heteroarylgruppe; eine Amidgruppe -C(=O)-NH-R⁶ oder eine Estergruppe -C(=O)-O-R⁶ stehen;
ferner, R⁵ Oxo sein kann, wenn die Bindung zwischen R⁵ und dem Kohlenstoff, an den R⁵ gebunden ist, eine Doppelbindung ist,
ferner, unter Beibehaltung der oben definierten Substituenten für R und R', die Gruppen R² und R³ oder R^{3a}, R³ oder R^{3a} und R⁴, R⁴ und R⁵ vereinigt sein können, um gemeinsam mit den benachbarten Kohlenstoffatomen zur Cyclohexanon-Gruppe, an der sie sich befinden, einen an das dasselbe Cyclohexanon kondensierten Ring mit 5, 6 oder 7 Atomen zu bilden;
- R⁶ für eine Alkylgruppe, eine Cycloalkylgruppe, eine Phenylgruppe, eine Arylgruppe oder eine Heteroarylgruppe steht;
- R⁷ steht für: -H; -OH; -NH₂; -NO₂; -SO₂CH₃; ein Halogenatom; eine Alkylgruppe; eine Alkoxygruppe; eine Cycloalkylgruppe; eine Phenylgruppe oder eine Arylgruppe,
wobei nicht umfasst ist die Verbindung der Formel I, in dem R ortho-NO₂ ist, R¹ para-CF₃ ist, R² = R³ = R^{3a} = R⁴ und H ist und R⁵ Oxo ist, wenn die Bindung zwischen R⁵ und dem Kohlenstoff, an dem R⁵ gebunden ist, eine Doppelbindung (bekannt als Nitisinon) ist.

2. Verbindung der Formel I zur Verwendung nach Anspruch 1, worin:
- das Halogenatom Cl oder Br ist und/oder;
- die Alkylgruppe eine Kohlenstoffkette umfasst, die von 1 bis 10 Kohlenstoffatomen enthält, wobei die Kette linear oder verzweigt, gesättigt oder ungesättigt, mit einer oder mehreren Doppelbindungen und/oder einer oder mehreren Dreifachbindungen ist, wobei die Kette unsubstituiert oder mit einem oder mehreren Heteroatomen wie O, S oder N, oder mit einem oder mehreren Halogenatomen, oder mit einer oder mehreren Aryl- oder Heteroarylgruppen und/oder substituiert ist;
- die Haloalkylgruppe CF₃, CHF₂, CH₂F ist und/oder;
- die Cycloalkylgruppe 3 bis 8 Kohlenstoffatome umfasst, die Cycloalkylgruppe bevorzugt Cyclopropyl, Cyclopentyl oder Cyclohexyl ist und/oder;
- die Heteroarylgruppe Pyrrol, Furan, Thiophen, Oxazol, Isoxazol, 3-Methylisoxazol, Pyrazol, Imidazol oder Pyridin ist.

3. Verbindung der Formel I zur Verwendung nach Anspruch 1 oder 2, worin:
- R H, *ortho-Chlor* oder *ortho-NO₂* ist;
- R¹ H, *para-Cl, para-SO₂CH₃, para-NO₂, para-CF₃* ist;
- R² H, Cl, OH, Phenyl, 3-Methlylisoxazol-6-yl ist;
- R³ e R^{3a} H oder CH₃ sind;
- R⁴ H, OH, Cl, COOCH₃ ist und
- R⁵ H oder Oxo ist, wenn die Bindung zwischen R⁵ und dem Kohlenstoff, an den R⁵ gebunden ist, eine Doppelbindung ist.

4. Verbindung der Formel I zur Verwendung nach Anspruch 1 oder 2, wobei die Verbindung ausgewählt ist aus der Gruppe bestehend aus:
- 2-[2-Chlor-4-methylsulfonyl-3-(2,2,2-trifluoretoxyimethyl)benzoyl]-4,6-dihydroxy-cyclohexan-1,3-dion, Verbindung **2:** 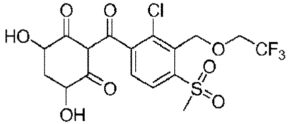
- 2-[2-Chlor-4-methylsulfonyl-3-(2,2,2-trifluoretoxymethyl)benzoyl]cyclohexan-1,3-dion, bekannt als Tembotrion, Verbindung **3:** 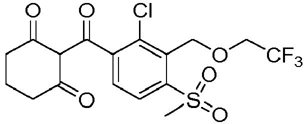
- 2-(4-Methylsulfonyl-2-nitrobenzoyl)-cyclohexan-1,3-dion, bekannt als Mesotrion, Verbindung **4:** 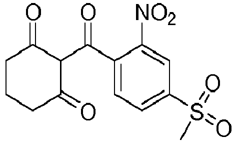
- 2-(2-Chlor-4-nitro-benzoyl)-5,5-dimethyl-cyclohexan-1,3-dion, Verbindung **6:** 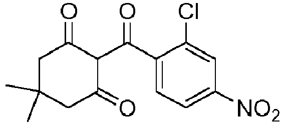
- 2,2-Dimethyl-5-(4-nitrobenzoyl)-4,6-dioxycyclohexan methyl carboxylat, Verbindung **7:** 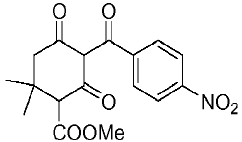
- 2-Benzoyl-4-chlor-5,5-dimethyl-cyclohexan-1,3-dion, Verbindung **8:** 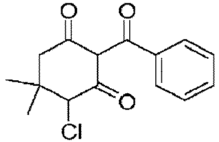
- 2-Benzoyl-5,5-dimethyl-cyclohexan-1,3-dion, Verbindung **9:** 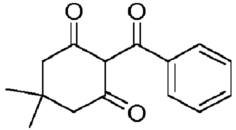
- 2-(4-Chlorbenzoyl)-5,5-dimethyl-4-(3-methylisoxazol-5-yl)-cyclohexan-1,3-dion, Verbindung **10:** 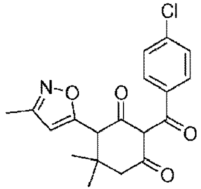
- 2-Benzoyl-4,6-dichlor-5,5-dimethyl-cyclohexan-1,3-dion, Verbindung **11:** 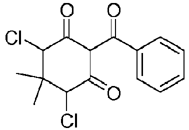
- 5-Benzoyl-2,2-dimethyl-4,6-dioxacyclohexan methyl carboxylat, Verbindung **12:** 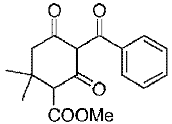
- 2-(2,4-Dichlorbenzoyl)-4-phenyl-cyclohexan-1,3-dion, Verbindung **13:** 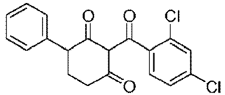
- 2-(2-Chlor-4-trifluormethylbenzoyl)-5,5-dimethylcyclohexan-1,3-dion, Verbindung **14:** 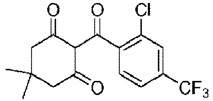
- 2-(2-Nitro-4-trifluormethylbenzoyl)-5,5-dimethylcyclohexan-1,3-dion, Verbindung **15:**

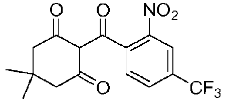

5. Verbindung der Formel I zur Verwendung nach Anspruch 1 oder 4, wobei die Verbindung ausgewählt ist aus der Gruppe bestehend aus:

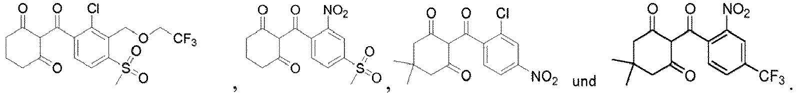

6. Verbindung der Formel I oder II zur Verwendung nach einem der Ansprüche 1 bis 5, wobei die Erkrankung, die **gekennzeichnet ist durch** die Akkumulation von HGA, Alkaptonurie ist.

7. Verbindung der Formel I oder II zur Verwendung nach einem der Ansprüche 1 bis 5, wobei die Erkrankung, die **gekennzeichnet ist durch** die Akkumulation von Succinylacetoacetat und Succinylaceton, Tyrosinämie Typ I ist.

8. Verbindung der Formel I oder II zur Verwendung nach einem der Ansprüche 1 bis 7 in Kombination mit einer therapeutischen Intervention.

9. Verbindung der Formel I oder II zur Verwendung nach Anspruch 8, wobei die therapeutische Intervention ausgewählt ist aus der Gruppe bestehend aus: proteinarmer Diät, Verabreichung von 4-HPPD-Inhibitor, Antioxidantien, Schmerzmitteln, entzündungshemmenden Medikamenten, Physiotherapie, Gelenkersatz.

10. Verbindung der Formel I oder II zur Verwendung nach Anspruch 8 oder 9, wobei die weitere therapeutische Intervention Nitisinon ist.

11. Wobei eine Verbindung der Formel I ist

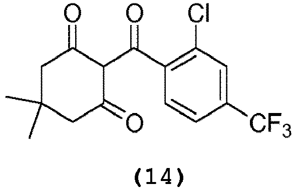

12. Verbindung nach Anspruch 11 in Kombination mit einer therapeutischen Intervention, wobei die therapeutische Intervention bevorzugt ausgewählt ist aus der Gruppe bestehend aus: proteinarmer Diät, Verabreichung eines 4-HPPD-Inhibitors, Antioxidantien, Schmerzmitteln, entzündungshemmenden Medikamenten, Physiotherapie, Gelenkersatz, wobei bevorzugt die weitere therapeutische Intervention Nitisinon ist.

13. Pharmazeutische Zusammensetzung, umfassend die Verbindung **(14)** oder **(15),** wie in Anspruch 4 definiert, und einen pharmazeutisch geeigneten Exzipienten.

14. Pharmazeutische Zusammensetzung, umfassend mindestens eine Verbindung der Formel I oder II, wie in einem der Ansprüche 1 bis 5 definiert, und einen pharmazeutisch geeigneten Exzipienten zur Verwendung bei der Vorbeugung und/oder Behandlung einer Erkrankung, die **gekennzeichnet ist durch** eine Akkumulation von HGA oder von Succinylacetoacetat und Succinylaceton, wobei ferner die pharmazeutische Zusammensetzung bevorzugt ein therapeutisches Mittel umfasst, wobei bevorzugt das therapeutische Mittel Nitisinon ist.

## Revendications

1. Composé de formule I ou II pour son utilisation dans la prévention et/ou le traitement d'une maladie **caractérisée par** une accumulation d'acide homogentisique (HGA) ou d'acétoacétate de succinyle et de succinylacétone : 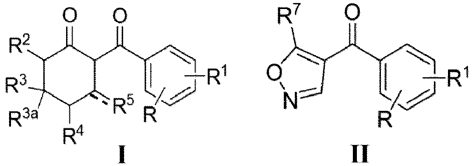 dans lequel
--- représente la présente ou l'absence d'une liaison ;
- R et R¹ sont identiques ou différents et indépendants l'un de l'autre, en position ortho, méta ou para, et représentent -H ; -OH ; -NH₂ ; -NO₂ ; -SO₂CH₃ ; un atome d'halogène ; un groupe alkyle ; un groupe halogénoalkyle ; un groupe alcoxy -O-alkyle ; un groupe cycloalkyle ; un groupe phényle ou un groupe aryle ;
- R², R³, R^{3a}, R⁴ et R⁵ sont identiques ou différents et indépendants les uns des autres, et représentent -H ;
- OH ; -NH₂ ; -NO₂ ; -SO₂CH₃ ; un atome d'halogène ; un groupe alkyle ; un groupe carbonyle ; un groupe cycloalkyle ; un groupe alcoxy ; un groupe phényle ; un groupe aryle, ou un groupe hétéroaryle ; un groupe amide
- C(=O)-NH-R⁶ ou un groupe ester -C(=O)-O-R⁶ ; en outre, R⁵ peut être oxo lorsque la liaison entre R⁵ et le carbone auquel R⁵ est lié est une double liaison,
en outre, en conservant les substituants définis ci-dessus pour R et R¹, les groupes R² et R³ ou R^{3a}, R³ ou R^{3a} et R⁴, R⁴ et R⁵ peuvent être fusionnés pour former, conjointement aux atomes de carbone adjacents au groupe cyclohexanone sur lequel ils se trouvent, un cycle avec 5, 6 ou 7 atomes condensés sur le même cyclohexanone ;
- R⁶ représente un groupe alkyle, un groupe cycloalkyle, un groupe phényle, un groupe aryle ou un groupe hétéroaryle ;
- R⁷ représente : -H ; -OH ; -NH₂ ; -NO₂ ; -SO₂CH₃ ; un atome d'halogène ; un groupe alkyle ; un groupe alcoxy ; un groupe cycloalkyle ; un groupe phényle ou un groupe aryle,
dans lequel le composé de formule I dans lequel R est ortho-NO₂, R¹ est para-CF₃, R² = R³ = R^{3a} = R⁴ et est H et R⁵ est oxo lorsque la liaison entre R⁵ et le carbone auquel R⁵ est lié est une double liaison (connu comme une nitisinone) n'est pas inclus.

2. Composé de formule I pour son utilisation selon la revendication 1 dans lequel :
- l'atome d'halogène est Cl ou Br et/ou ;
- le groupe alkyle comprend une chaîne carbonée qui contient de 1 à 10 atomes de carbone, ladite chaîne étant linéaire ou ramifiée, saturée ou insaturée, avec une ou plusieurs doubles liaisons et/ou une ou plusieurs triples liaisons, ladite chaîne étant non substituée ou substituée avec un ou plusieurs hétéroatomes tels que O, S, ou N, ou avec un ou plusieurs atomes d'halogène, ou avec un ou plusieurs groupes aryle ou hétéroaryle et/ou ;
- le groupe halogénoalkyle est CF₃, CHF₂, CH₂F et/ou ;
- le groupe cycloalkyle comprend de 3 à 8 atomes de carbone, de préférence le groupe cycloalkyle est un cyclopropyle, cyclopentyle ou cyclohexyle et/ou ;
- le groupe hétéroaryle est un pyrrole, un furane, un thiophène, un oxazole, un isoxazole, un 3-méthylisoxazole, un pyrazole, un imidazole ou une pyridine.

3. Composé de formule I pour son utilisation selon la revendication 1 ou 2 dans lequel :
- R est H, *ortho*-chloro ou *ortho*-NO₂ ;
- R¹ est H, *para*-Cl, *para*-SO₂CH₃, *para*-NO₂, *para*-CF₃,
- R² est H, Cl, OH, phényle, 3-méthylisoxazol-6-yl ;
- R³ et R^{3a} sont H ou CH₃ ;
- R⁴ est H, OH, Cl, COOCH₃ et
- R⁵ est H ou oxo lorsque la liaison entre R⁵ et le carbone auquel R⁵ est lié est une double liaison.

4. Composé de formule I pour son utilisation selon la revendication 1 ou 2 dans lequel le composé est sélectionné dans le groupe constitué des :
- 2-[2-chloro-4-méthylsulfonyl-3-(2,2,2-trifluoroétoxyiméthyl)benzoyl]-4,6-dihydroxy-cyclohexane-1,3-dione, composé 2 : 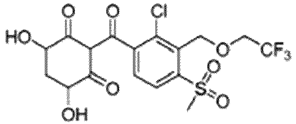
- 2-[2-chloro-4-méthylsulfonyl-3-(2,2,2-trifluoroétoxyméthyl)benzoyl]cyclohexane-1,3-dione, connue comme la tembotrione, composé 3 : 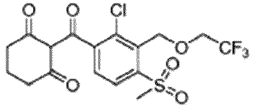
- 2-(4-méthylsulfonyl-2-nitrobenzoyl)-cyclohexane-1,3-dione, connue comme la mésotrione, composé 4 : 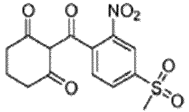
- 2-(2-chloro-4-nitro-benzoyl)-5,5-diméthyl-cyclohexane-1,3-dione, composé 6 : 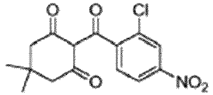
- méthyl carboxylate de 2,2-diméthyl-5-(4-nitrobenzoyl)-4,6-dioxycyclohexane, composé 7 : 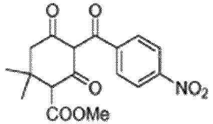
- 2-benzoyl-4-chloro-5,5-diméthyl-cyclohexane-1,3-dione, composé 8 : 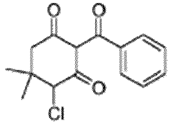
- 2-benzoyl-5,5-dimethyl-cyclohexane-1,3-dione, composé 9 : 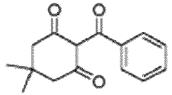
- 2-(4-chlorobenzoyl)-5,5-diméthyl-4-(3-méthyl-isoxazol-5-yl)-cyclohexane-1,3- dione, composé 10 : 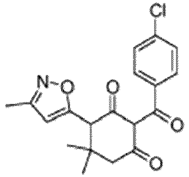
- 2-benzoyl-4,6-dichloro-5,5-diméthyl-cyclohexane-1,3-dione, composé 11 : 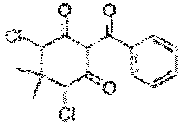
- méthyl carboxylate de 5-benzoyl-2,2-diméthyl-4,6-dioxacyclohexane, composé 12 : 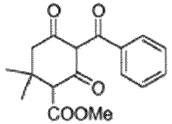
- 2-(2,4-dichlorobenzoyl)-4-phényl-cyclohexane-1,3-dione, composé 13 : 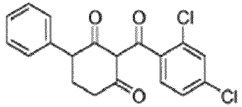
- 2-(2-chloro-4-trifluorométhylbenzoyl)-5,5-diméthyl-cyclohexane-1,3-dione, composé 14 : 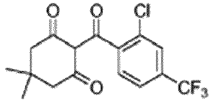
- 2-(2-nitro-4-trifluorométhylbenzoyl)-5,5-diméthyl-cyclohexane-1,3-dione, composé 15 :

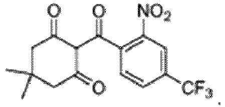

5. Composé de formule I pour son utilisation selon la revendication 1 ou 4 dans lequel le composé est sélectionné dans le groupe constitué de :

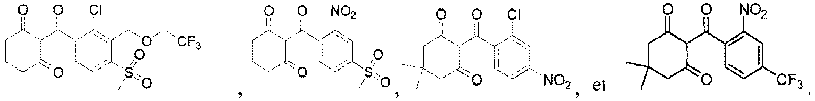

6. Composé de formule I ou II pour son utilisation selon l'une quelconque des revendications 1 à 5 dans lequel la maladie **caractérisée par** l'accumulation d'HGA est l'alcaptonurie.

7. Composé de formule I ou II pour son utilisation selon l'une quelconque des revendications 1 à 5 dans lequel la maladie **caractérisée par** l'accumulation d'acétoacétate de succinyle et de succinylacétone est la tyrosinémie de type I.

8. Composé de formule I ou II pour son utilisation selon l'une quelconque des revendications 1 à 7 en combinaison avec une intervention thérapeutique.

9. Composé de formule I ou II pour son utilisation selon la revendication 8 dans lequel l'intervention thérapeutique est sélectionnée dans le groupe constitué de : un régime faible en protéines, l'administration d'un inhibiteur de 4-HPPD, d'anti-oxydants, d'analgésiques, de médicaments anti-inflammatoires, d'une physiothérapie, d'une prothèse d'articulation.

10. Composé de formule I ou II pour son utilisation selon la revendication 8 ou 9 dans lequel l'intervention thérapeutique supplémentaire est la nitisinone.

11. Composé de formule I étant

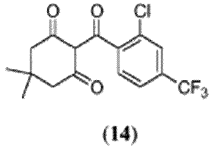

12. Composé selon la revendication 11 en combination avec une intervention thérapeutique, de préférence l'intervention thérapeutique est sélectionnée dans le groupe constitué de : un régime faible en protéines, l'administration d'un inhibiteur de 4-HPPD, d'antioxydants, d'analgésiques, de médicaments anti-inflammatoires, d'une physiothérapie, d'une prothèse d'articulation, de préférence l'intervention thérapeutique supplémentaire est la nitisinone.

13. Composition pharmaceutique comprenant le composé (14) ou (15) selon la revendication 4 et un vecteur pharmaceutiquement acceptable.

14. Composition pharmaceutique comprenant au moins un composé de formule I ou II selon l'une quelconque des revendications 1 à 5 et un vecteur pharmaceutiquement acceptable pour son utilisation dans la prévention et/ou le traitement d'une maladie **caractérisée par** une accumulation d'HGA ou d'acétoacétate de succinyle et de succinylacétone, de préférence la composition pharmaceutique comprend en outre un agent thérapeutique, de préférence l'agent thérapeutique est la nitisinone.
